# EUROPEAN PATENT APPLICATION

(11) **EP 2 138 094 A1**
(43) Date of publication of application: **30.12.2009**
(21) Application number: 07737101.1
(22) Date of filing: 24.04.2007
(51) Int. Cl.: A61B 5/00

(54) **BIOLOGICAL INFORMATION DETECTION DEVICE**

(71) Applicant: FiberTech CO., LTD., 3-21, Kandanishiki-cho Chiyoda-ku Tokyo 101-0054 (JP)
(72) Inventor: TAMURA, Yasuhiro, Yashio-shi Saitama 340-0811 (JP)
(74) Representative: Roberts, Peter David
(86) International application number: PCT/JP2007/000444
(87) International publication number: WO 2008/136050

(57) **Abstract**

A biological information detection apparatus includes a sensor, a memory unit, and a control unit. The sensor is worn by a subject and continuously detects the biological information of the subject. The memory unit stores biological information detected by the sensor. The control unit takes control so that a predetermined part of the biological information stored in the memory unit is transmitted to a management apparatus on a regular basis. The control unit takes control so that, when the biological information detected by the sensor exceeds a predetermined reference value, the amount of information, which is larger than the amount of information regularly transmitted, is transmitted to the management apparatus.

## Description

### [Technical Field]

The present invention relates to a biological information detection apparatus that detects biological information of a human body by using a sensor and transmits the information to the outside.

### [ Background Art]

Various apparatuses and systems have been suggested for supporting the care and nursing of the elderly and the sick. In particular, there is a strong need for efficient general care and healthcare for the elderly due to the rapid aging of the population amid extremely low birthrates. As one of such apparatuses, an apparatus has been suggested that acquires biological information from a sensor worn on the human body of a receiver of general care or of nursing-care receiver and transmits the biological information to a management apparatus via a wired or wireless line (e.g. patent document 1 and patent document 2).
[ Patent document 1] JP 2006-297068
[ Patent document 2] JP 2006-263305

### [ Disclosure of Invention]

### [ Technical Problem]

An apparatus carrying a sensor as described above needs to be worn on or attached to a human body. Thus, a configuration is desirable in which a power supply line or a communication line is not connected so that the freedom of the wearer is unrestricted. In other words, a battery-driven configuration carrying a wireless communication function is desirable. However, a heavy-load process performed in a battery-driven configuration requires frequent replacement of the battery, which is troublesome and not economical. On the other hand, an overly simplified process may result in a situation where necessary information is not transmitted to a management apparatus, preventing the prompt provision of a necessary treatment.

In this background, a general purpose of the present invention is to provide a biological information detection apparatus that provides high-quality information to an external apparatus while suppressing power consumption.

### [Means for Solving the Problem]

A biological information detection apparatus of the present invention comprises: a sensor to be worn by a subject operative to continuously detect the biological information of the subject; a memory unit operative to store the biological information detected by the sensor; and a control unit operative to take control so that a predetermined part of the biological information stored in the memory unit is transmitted to a management apparatus on a regular basis, wherein the control unit takes control so that, when the biological information detected by the sensor exceeds a predetermined reference value, the amount of information, which is larger than the amount of information regularly transmitted, is transmitted to the management apparatus.

Optional combinations of the aforementioned constituting elements, and implementations of the invention in the form of methods, systems, recording mediums, and computer programs may also be practiced as additional modes of the present invention.

### [Advantageous Effects of Invention]

The present invention provides high-quality information to an external apparatus while suppressing power consumption.

### [Brief Description of Drawings]

Fig. 1 is a diagram showing the configuration of a biological information management system according to the embodiment 1 of the present invention;
Fig. 2 is a diagram showing the configuration of a biological information detection apparatus according to the embodiment 1;
Fig. 3 is a diagram showing the configuration of a management apparatus according to the embodiment 1;
Fig. 4 is a timing chart explaining an operation example of the biological information detection apparatus according to the embodiment 1;
Fig. 5 is a flowchart explaining the operation of the biological information detection apparatus according to the embodiment 1;
Fig. 6 is a diagram showing the configuration of the biological information management system according to the embodiment 2 of the present invention;
Fig. 7 is a diagram showing the configuration of the biological information detection apparatus according to the embodiment 2; and
Fig. 8 is a flowchart explaining the operation of the biological information detection apparatus according to the embodiment 2.

### [Reference Signs List]

- 10: control unit
- 20: sensor unit
- 22: body temperature sensor
- 24: heartbeat sensor
- 25: pulse sensor
- 26: acceleration sensor
- 30: memory unit
- 32: GPS reception unit
- 35: clock
- 40: communication unit
- 100, 150: biological information detection apparatus
- 200: management apparatus
- 300: relay apparatus
- 500, 550: biological information management system

### [Detailed Description of the Invention]

### (Embodiment 1)

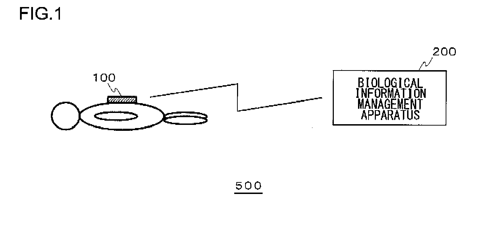

Fig. 1 is a diagram showing the configuration of a biological information management system 500 according to the embodiment 1 of the present invention. The biological information management system 500 is provided with a biological information detection apparatus 100 and a management apparatus 200. The biological information detection apparatus 100 is used by being worn on a human body. The embodiment 1 is explained in the following by using an example where a patient or a receiver of general care (hereinafter, referred to as a subject), who lies on a bed in a medical facility or a care facility, wears the biological information detection apparatus 100 as an example of a mode of use of the biological information detection apparatus 100. Therefore, the management apparatus 200 is preferably installed in a room where a healthcare provider or a care giver is assigned. Desirably, the management apparatus 200 is configured with a mobile terminal and carried by a healthcare provider.

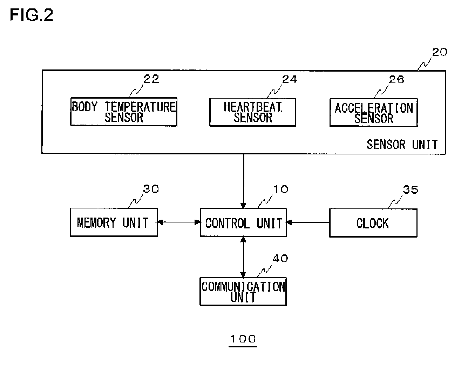

Fig. 2 is a diagram showing the configuration of a biological information detection apparatus 100 according to the embodiment 1. The biological information detection apparatus 100 carries a board provided with a processor and memory. The biological information detection apparatus 100 is provided with a control unit 10, a sensor unit 20, a memory unit 30, a clock 35, and a communication unit 40. Desirably, the biological information detection apparatus 100 is battery-driven so as not to require wirings. The sensor unit 20 is an integrated sensor unit including a body temperature sensor 22, a heartbeat sensor 24, and an acceleration sensor 26 and continuously detects the biological information of a subject. The continuous detection includes a mode where the detection occurs at short time intervals. For example, a mode is included where a body temperature is detected for every one minute. In the specification, the biological information includes not only physiological information such as a body temperature and a heart rate or a pulse rate, which is the beating rate per minute, but also physical information such as a posture.

The body temperature sensor 22 detects the body temperature or the surface temperature of a subject and outputs the detected temperature to the control unit 10. The heartbeat sensor 24 is provided with an electrode sheet, and the electrode sheet is attached to the chest region of the subject. The heartbeat 24 detects the heartbeat of the subject and outputs the detected heartbeat to the control unit 10. The acceleration sensor 26 detects the acceleration in the directions of two axes, the X axis and the Y axis, which are orthogonal to each other, or the acceleration in the directions of three axes additionally including the Z axis, and outputs the detected acceleration to the control unit 10.

The memory unit 30 stores various biological information detected by the sensor unit 20. The memory unit 30 has a predetermined memory area and may be configured with a loop buffer that overwrites data sequentially from first-stored data when the memory area has overflowed. It is assumed that the memory unit 30 is provided with an area capable of storing data for an interval of one hour. The control unit 10 sequentially writes the temperature and a heart rate in the memory area every one minute and overwrites, when the memory area is full, the data in the area in which the data from the previous hour has been written. This process allows the memory unit 30 to always keep the latest biological information for an hour.

The clock 35 provides the time to the control unit 10. The communication unit 40 transmits the information specified by the control unit 10 to the management apparatus 200 by using a predetermined wireless line. The communication unit 40 also receives a predetermined instruction from the management apparatus 200. For example, a packet communication network provided by a mobile service provider or the like can be used as the predetermined wireless line. When a LAN (Local Area Network) is installed in a medical facility or care facility and when the management apparatus 200 is connected to the LAN, the communication unit 40 may communicate with the management apparatus 200 by performing a short-distance wireless communication with an access point of the LAN.

The control unit 10 takes control so that a predetermined part of the biological information stored in the memory unit 30 is transmitted to the management apparatus 200 on a regular basis. For example, the control unit 10 continuously measures a heart rate by using a heartbeat sensor 24 and stores the heart rate in the memory unit 30 every one minute. The control unit 10 takes control so that only the latest heart rate information from the multiple heat rate information pieces stored in the memory unit 30 is transmitted to the management apparatus 200 every one hour. The body temperature sensor 22 can be processed in a similar manner. This allows for the suppression of the battery consumption. Both the information to be transmitted on a regular basis and the transmission interval may be set by default or may be set by a healthcare provider or a care giver via the management apparatus 200.

The control unit 10 takes control so that, when the biological information detected by the sensor unit 20 exceeds a predetermined reference value, the amount of information, which is larger than the amount of information regularly transmitted, is transmitted to the management apparatus 200. For example, the control is designed so that all the biological information that is stored in the memory unit 30 at the moment is transmitted to the management apparatus 200. The control unit 10 may take control so that, in addition to the part of the information set in advance, other types of information are transmitted. For example, when the detected biological information exceeds the predetermined reference value while the body temperature is being regularly transmitted as a part of the information, a heart rate may be additionally transmitted. When the predetermined reference value is exceeded, there is a high possibility of something out of the ordinary has happened to a subject. A detailed description will be given of an example of the predetermined reference value. Therefore, it is desirable to provide a healthcare provider or a care giver with as much information as possible for finding out what has happened.

The control unit 10 takes control so that, when the biological information detected by the sensor unit 20 exceeds a predetermined reference value, the interval of transmission to the management apparatus 200 becomes shorter. Alternatively, the control unit 10 takes control so that continuous transmission is carried out. For example, the transmission interval set to one hour is changed to one minute. The detection result may be transmitted as soon as it is acquired. This can result in continuous transmission. Such a process is also for the purpose of providing as much biological information to a healthcare provider or a care giver after the detection of an abnormal value exceeding a reference value.

As an example of a predetermined reference value, upper threshold and lower threshold are set for the detection of an abnormity in the heart rate, and upper threshold and lower threshold are set for the detection of an abnormity in the body temperature. For example, the upper threshold and the lower threshold of the heart rate are set to 100 and 60, respectively. A healthcare provider or a care giver can set the preferred reference value for each subject in consideration of the medical condition, the age, and the physical condition of the subject.

An acceleration component can be set as a predetermined reference value. For example, when a large acceleration component is detected from the acceleration sensor 26 worn by, for example, a bedridden patient, there is the possibility of some abnormal situations occurring such as the patient falling from his/her bed. The maximum acceleration component that can be detected for the normal movement of a bedridden patient is set as the predetermined reference value so that the abnormal situation can be confirmed by the outside such as from a nurse station.

Upon the receipt of a request for transmitting the predetermined information from the management apparatus 200, the control unit 10 takes control so as to specify the requested information from the biological information stored in the memory unit 30 and to transmit the information to the management apparatus 200. This process allows the management apparatus 200 to obtain the information needed by a healthcare provider or a care giver at an arbitrary time by efficiently using a bidirectional communication function.

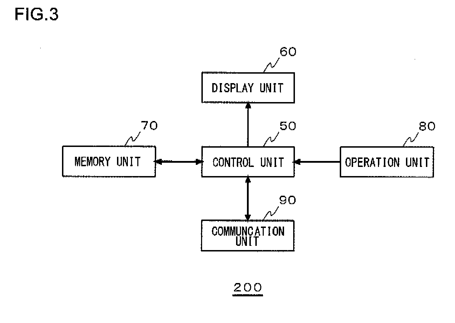

Fig. 3 is a diagram showing the configuration of the management apparatus 200 according to the embodiment 1. The management apparatus 200 is configured with a PC, a server, or a mobile terminal. The management apparatus 200 is provided with a control unit 50, a display unit 60, a memory unit 70, an operation unit 80, and a communication unit 90. The control unit 50 controls overall the management apparatus 200. The display unit 60 displays, for example, the biological information obtained from the biological information detection apparatus 100. The memory unit 70 makes a database of the biological information obtained from the biological information detection apparatus 100 and stores the information. The operation unit 80 makes a request to the biological information detection apparatus 100 for adding, changing, or deleting various settings or for the transmission of specific information upon the receipt of an operation from a healthcare provider or a care giver. The communication unit 90 communicates with a communication unit 40 of the biological information detection apparatus 100 in a bidirectional manner.

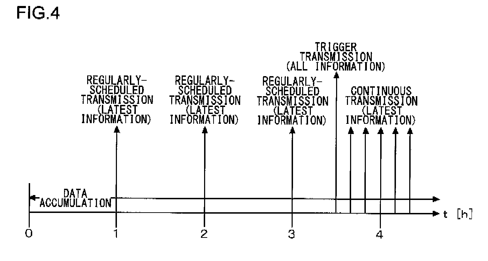

Fig. 4 is a timing chart explaining an operation example of the biological information detection apparatus 100 according to the embodiment 1. Upon the acquisition of the biological information detected by the sensor unit 20, the control unit 10 stores the information in the memory unit 30. The control unit 10 takes control so as to regularly transmit the biological information every hour. In this case, the control unit 10 takes control so as to transmit only the latest information from the biological information for an interval of one hour, which is stored in the memory unit 30. This process is repeated until an abnormal value is detected.

Upon the detection of an abnormal value, the control unit 10 takes control so as to trigger transmit the biological information to the management apparatus 200 by using the detection as a trigger. Fig. 4 shows an example of when an abnormal value is detected between three and four o'clock. In the case of the trigger transmission, the biological information for an interval of one hour, which is stored in the memory unit 30, is controlled so that all the information is transmitted. The biological information may be controlled so that only the biological information related to the abnormal value is fully transmitted. For example, when an abnormity in the heart rate is detected, control may be taken so that only the heart rate for an interval of one hour is transmitted. When the reference value is exceeded by a relatively small amount, the setting may be made so that not all the information but more than the amount of regularly-transmitted information is transmitted. For example, the biological information not for the interval of one hour but for an interval of 30 minutes may be transmitted.

Upon the detection of an abnormal value, the control unit 10 may take control so that not regularly-scheduled transmission but continuous transmission is carried out. When the reference value is exceeded by a relatively small amount, control may be taken so that the frequency of regularly-scheduled transmission is increased but not by so much as to be considered as continuous transmission.

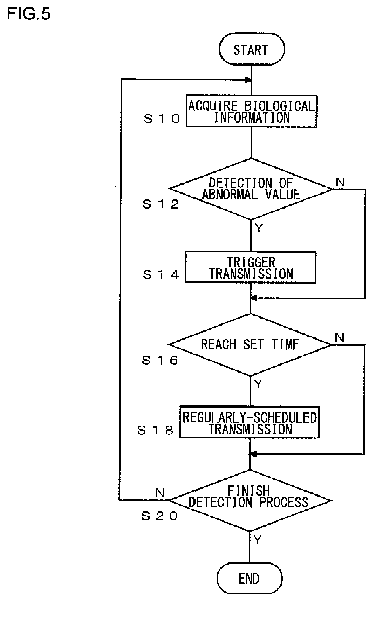

Fig. 5 is a flowchart explaining the operation of the biological information detection apparatus 100 according to the embodiment 1. The control unit 10 continuously acquires biological information from the sensor unit 20 and stores the information in the memory unit 30 (S10). When an abnormal value that exceeds a predetermined reference value is detected (Y in S12), the above-mentioned trigger transmission process is activated (S14). Until an abnormal value is detected (N in S12), the above-mentioned regularly-scheduled transmission is activated (S18) at the set time (Y in S16). While the detection process continues (N in S20), the step transits to the step S10 and a similar process is repeated. The above-mentioned continuous transmission process may be activated after the trigger transmission process is activated.

As described above, intermittent regularly-scheduled transmission of biological information while no abnormal value is produced allows for the throughput to be reduced compared to when biological information for the entire period of time is transmitted and thus allows the power consumption to be suppressed, according to the embodiment 1. When an abnormal value is detected, transmitting a great amount of information including history information allows for the maintenance of the quality of the information obtained by a management apparatus. Specific biological information can be acquired from the biological information detection apparatus 100 at an arbitrary time by the request from the management apparatus. Thus, the quality of the information obtained by the management apparatus is ensured from this aspect. Increasing the frequency of the transmission after the detection of an abnormal value responds to the request for the provision of much information when it is expected.

As the power consumption is reduced, the frequency of replacing the battery decreases in a battery-driven apparatus in which a sensor is worn on a human body. The apparatus according to the embodiment 1 can reduce the power consumption, and the frequency of replacing the battery is thus decreased. Also, the time required for the transmission of the biological information can be shortened, and the communication charge can thus be lowered. A battery-driven wireless communication apparatus allows for the acquisition of valid information with almost no restriction of a subject's freedom.

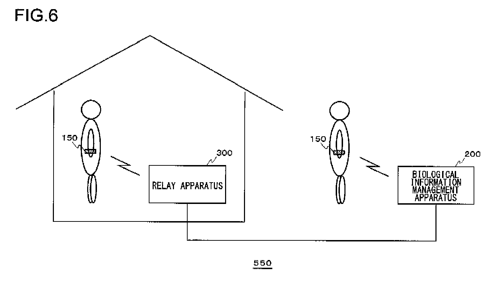

(Embodiment 2) Fig. 6 is a diagram showing the configuration of a biological information management system 550 according to the embodiment 2 of the present invention. The biological information management system 550 is provided with a biological information detection apparatus 150, a management apparatus 200, and a relay apparatus 300. The relay apparatus 300 is configured with a home server installed indoors and functions as a gateway apparatus for the Internet. The management apparatus 200 is configured with a PC, a server, or a mobile terminal such as a mobile phone. When configured with a mobile terminal, the management apparatus 200 may communicate with the biological information detection apparatus 150 directly or via an Internet server. The management apparatus 200 is provided with a feature similar to that of the embodiment 1.

The biological information detection apparatus 150 is used by being worn on a human body. The embodiment 2 is explained in the following by using an example where an elderly person who stays at home alone wears the biological information detection apparatus 150 as an example of a mode of use of the biological information detection apparatus 150. The portable management apparatus 200 allows the spouse or child of the elderly person to acquire the biological information from outside of the home or from a separate home.

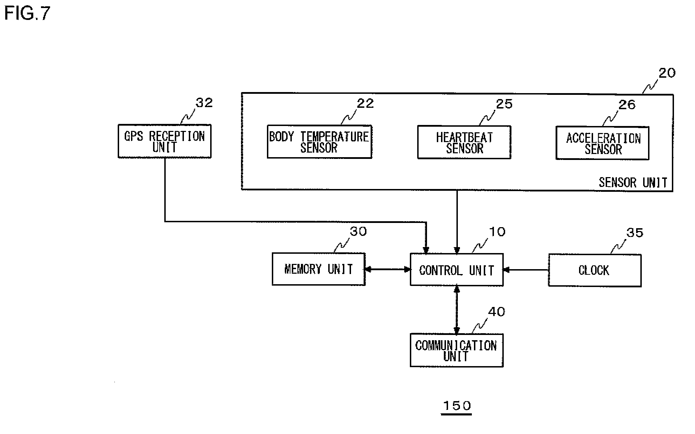

Fig. 7 is a diagram showing the configuration of the biological information detection apparatus 150 according to the embodiment 2. The biological information detection apparatus 150 carries a board provided with a processor and memory. The biological information detection apparatus 150 is provided with the control unit 10, the sensor unit 20, the memory unit 30, a GPS reception unit 32, the clock 35, and the communication unit 40. The biological information detection apparatus 150 is battery-driven. The sensor unit 20 is an integrated sensor unit including the body temperature sensor 22, a pulse sensor 25, and the acceleration sensor 26 and continuously detects the biological information of a subject. The sensor unit 20 is worn on the subject's arm like a wrist watch.

The body temperature sensor 22 detects the body temperature or the surface temperature of a subject and outputs the detected temperature to the control unit 10. The pulse sensor 25 detects the pulse of the subject and outputs the detected pulse to the control unit 10. The acceleration sensor 26 detects acceleration in the direction of two axes or three axes and outputs the detected acceleration to the control unit 10.

The memory unit 30 stores various biological information detected by the sensor unit 20. The GPS reception unit 32 receives radio wave information from multiple GPS satellites and outputs the information to the control unit 10. The clock 35 provides the time to the control unit 10.

The communication unit 40 transmits the information specified by the control unit 10 to the relay apparatus 300 or the management apparatus 200 by using a predetermined wireless line. The communication unit 40 also receives a predetermined instruction from the relay apparatus 300 or the management apparatus 200. The communication unit 40 communicates with the relay apparatus 300 by using an infrared ray or a 300MHz or 900MHz band radio wave. When directly communicating with the management apparatus 200, the communication unit 40 uses a packet communication network or the like.

The control unit 10 takes control so that the biological information detected by the sensor unit 20 is transmitted to the external management apparatus 200 via the relay apparatus 300 by transmitting the information wirelessly to the relay apparatus 300 installed indoors when a communication channel to the relay apparatus 300 is established. In contrast, the control unit 10 takes control so that the biological information is transmitted to the management apparatus 200 via a wireless communication network such as a packet communication network without going through the relay apparatus 300 when the communication channel to the relay apparatus 300 is not established.

The biological information detection apparatus 150 and the relay apparatus 300 can communicate with each other by using the above-stated communication means when the distance between the two is about 300m or less. Thus, the communication channel to the relay apparatus 300 is normally established when the subject stays indoors. On the other hand, the communication channel to the relay apparatus 300 cannot be established when the subject goes outside. Conversely, when the communication channel to the relay apparatus 300 cannot be established, it is assumed at the management apparatus 200 side that the subject is most likely outside.

The control unit 10 takes control so that the positional information of the subject wearing the biological information detection apparatus 150 is generated and transmitted to the management apparatus 200 along with the biological information, based on the radio wave information received by the GPS reception unit 32, when the communication channel to the relay apparatus 300 cannot be established. More specifically, the control unit 10 calculates the latitude and longitude of the position of the subject wearing the biological information detection apparatus 150 based on the radio wave information including multiple time information pieces that are received by the GPS reception unit 32.

The time at which the biological information stored in the memory unit 30 is transmitted to the management apparatus 200 may be determined by using, but not limited to, the method shown in the embodiment 1.

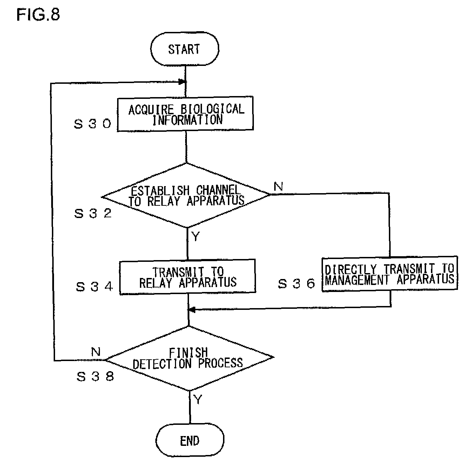

Fig. 8 is a flowchart explaining the operation of the biological information detection apparatus 150 according to the embodiment 2. The control unit 10 first acquires the biological information from the sensor unit 20 and stores the information in the memory unit 30 (S30). When the communication channel to the relay apparatus 300 is established (Y in S32), the control unit 10 takes control so that the biological information is transmitted to the management apparatus 200 via the relay apparatus 300 (S34). When the communication channel to the relay apparatus 300 cannot be established (N in S32), the control unit 10 takes control so that the biological information is directly transmitted to the management apparatus 200 by using, for example, a packet communication network (S36). While the detection process continues (N in S38), the step transits to the step S30 and a similar process is repeated.

As described above, the biological information can be transmitted to a management apparatus by using a wireless communication network even when a communication channel to a relay apparatus installed indoors cannot be established, according to the embodiment 2. In other words, the biological information can be transmitted without any restrictions regarding the position of a subject, and whether or not an abnormal condition has occurred with a subject can be still checked even when the subject goes outside. Also, the communication charge can be lowered by using the relay apparatus when the communication channel to the relay channel is established.

Also, when the communication channel to the relay channel cannot be established and when the subject is assumed to be outside, the position of the subject can be followed by activating a GPS feature. The unnecessary process of computing the positional information while the subject stays indoors can be avoided by activating the GPS function only when the subject goes outside. This in combination with the transmission process of the embodiment 1 allows for a further efficient process, and the power consumption can thus be further reduced.

Described above is an explanation of the present invention based on the embodiments. The embodiments are intended to be illustrative only and it will be obvious to those skilled in the art that various modifications to constituting elements and processes could be developed and that such modifications are also within the scope of the present invention.

For example, a reference value is set for the determination of the occurrence of any abnormity in the embodiment 1. Additionally, the degree of the abnormity may be determined by setting multiple reference values in stages. The subsequent process can be changed according to the degree. For example, control can be taken by providing reference values in stages, e.g., 37°C, 38°C, and 39°C, for the determination of an abnormity in the body temperature in such a manner as follows: the one-hour interval of a regularly-scheduled transmission is changed to 15 minutes when the body temperature exceeds 37°C; a similar process as that of the embodiment 1 is performed when the body temperature exceeds 38°C; and a request is made for outputting a warning tone to the management apparatus 200 when the body temperature exceeds 39°C. This allows for the further optimization of the balance between the power consumption and the provision of information.

In the embodiment 2, the step count may be acquired by analyzing the component of acceleration obtained by the acceleration sensor. Furthermore, the total daily consumed calories may be calculated by multiplying the step count by the calories consumed for unit walking movement. Storing such information together in combination allows the information to be used for the health management of a subject.

Sensors installed on the sensor unit 20 described in the embodiments are not limited to those mentioned above. Various sensors such as a humidity sensor, a radiation sensor, or an infrared radiation sensor can be installed on the sensor unit 20.

### [Industrial applicability]

The present invention can be applied to the field related to a biological information detection apparatus that detects the biological information of a human body by using a sensor and transmits the information to the outside.

## Claims

1. A biological information detection apparatus comprising:
a sensor to be worn by a subject operative to continuously detect the biological information of the subject;
a memory unit operative to store the biological information detected by the sensor; and
a control unit operative to take control so that a predetermined part of the biological information stored in the memory unit is transmitted to a management apparatus on a regular basis, wherein
the control unit takes control so that, when the biological information detected by the sensor exceeds a predetermined reference value, the amount of information, which is larger than the amount of information regularly transmitted, is transmitted to the management apparatus.

2. The biological information detection apparatus according to claim 1, wherein
the memory unit has a predetermined memory area and is a loop buffer that overwrites data sequentially from first-stored data when the memory area has overflowed, and
the control unit takes control so that, when the biological information detected by the sensor exceeds a predetermined reference value, all the information stored in the memory unit is transmitted to the management apparatus.

3. A biological information detection apparatus comprising:
a sensor to be worn by a subject operative to continuously detect the biological information of the subject;
a memory unit operative to store the biological information detected by the sensor; and
a control unit operative to take control so that a predetermined part of the biological information stored in the memory unit is transmitted to a management apparatus on a regular basis, wherein
the control unit takes control so that, when the biological information detected by the sensor exceeds a predetermined reference value, the interval of transmission to the management apparatus becomes shorter.

4. The biological information detection apparatus according to any one of claims 1 through 3, wherein the control unit takes control, upon the receipt of a request for transmitting predetermined information from the management apparatus, so as to specify the predetermined information from the biological information stored in the memory unit and transmit the information to the management apparatus.

5. A biological information detection apparatus comprising:
a sensor to be worn by a subject operative to detect the biological information of the subject; and
a control unit operative to take control so that the biological information detected by the sensor is transmitted, by transmitting the information wirelessly to a relay apparatus, to an external management apparatus via the relay apparatus, wherein
the control unit takes control so that, when a communication channel to the relay apparatus is not established, the biological information is transmitted to the management apparatus via a predetermined wireless communication network without going through the relay apparatus.

6. The biological information detection apparatus according to claim 5 further comprising:
a GPS reception unit operative to receive radio wave information from a plurality of GPS satellites, wherein
the control unit takes control so that, when the communication channel to the relay apparatus cannot be established, the positional information of a subject wearing the sensor is generated and transmitted to the management apparatus along with the biological information, based on the radio wave information received by the GPS reception unit.

7. The biological information detection apparatus according to any one of claims 1 through 6, wherein the biological information detection apparatus is battery-driven.

## Amended claims

### Amended claims under Art. 19.1 PCT

1. (Amended) A biological information detection apparatus comprising:
a sensor to be worn by a subject operative to continuously detect the biological information of the subject;
a memory unit operative to store the biological information detected by the sensor; and
a control unit operative to take control so that a predetermined part of the biological information stored in the memory unit is transmitted to a management apparatus on a regular basis, wherein
the control unit determines the degree of an abnormity by comparing the biological information detected by the sensor and a plurality of reference values in stages and performs a different process according to the degree.

2. (Amended) The biological information detection apparatus according to claim 1, wherein the control unit shortens the interval of transmission to the management apparatus as the degree of the abnormity increases.

3. (Amended) The biological information detection apparatus according to claim 1, wherein the control unit transmits more biological information to the management apparatus as the degree of the abnormity increases.

4. (Amended) The biological information detection apparatus according to any one of claims 1 through 3, wherein the control unit takes control so that a warning tone is output from the management apparatus when the reference value, among the plurality of reference values, for the highest degree of the abnormity is exceeded.

5. (Amended) The biological information detection apparatus according to claim 1, wherein the control unit takes control so that the biological information detected by the sensor is transmitted, by transmitting the information wirelessly to a relay apparatus, to an external management apparatus via the relay apparatus and takes control so that, when a communication channel to the relay apparatus is not established, the biological information is transmitted to the management apparatus via a predetermined wireless communication network without going through the relay apparatus.

6. (Amended) The biological information detection apparatus according to claim 5 further comprising: a GPS reception unit operative to receive radio wave information from a plurality of GPS satellites, wherein the control unit takes control so that, when the communication channel to the relay apparatus cannot be established, the positional information of a subject wearing the sensor is generated and transmitted to the management apparatus along with the biological information, based on the radio wave information received by the GPS reception unit.

7. (Amended) The biological information detection apparatus according to any one of claims 1 through 6, wherein the biological information detection apparatus is battery-driven.
